# EUROPEAN PATENT SPECIFICATION

(11) **EP 2 590 568 B1**
(45) Date of publication and mention of the grant of the patent: **24.02.2016**
(21) Application number: 11755458.4
(22) Date of filing: 07.07.2011
(51) Int. Cl.: A61B 5/00, A63B 71/06, A61H 1/02, A63B 19/02, A61B 5/11, A61B 5/22, A63B 23/035, A63B 24/00, G09B 5/02, A63B 21/00

(54) **DEVICE FOR ASSESSING AND DISPLAY OF A DIFFERENCE IN VECTORS OF FORCES EXERCISED BY A PAIR OF ARMS OR LEGS**
VORRICHTUNG ZUR BEURTEILUNG UND ANZEIGE EINER DIFFERENZ IN VEKTOREN VON KRÄFTEN, DIE VON EIN PAAR ARMEN ODER BEINEN AUSGEÜBT WERDEN
DISPOSITIF D'ÉVALUATION ET D'AFFICHAGE D'UNE DIFFÉRENCE VECTORIELLE DE FORCES EXERCÉES PAR UNE PAIRE DE BRAS OU DE JAMBES

(30) Priority: 07.07.2010 SI 201000204
(43) Date of publication of application: 15.05.2013
(73) Proprietor: Kinestica D.O.O., 1000 Ljubljana (SI)
(72) Inventor: MIHELJ, Matjaz, 5295 Branik (SI); MUNIH, Marko, 1370 Logatec (SI); TRLEP, Matic, 4224 Gorenja vas (SI)
(74) Representative: Golmajer Zima, Marjanca
(86) International application number: PCT/SI2011/000037
(87) International publication number: WO 2012/005702

(56) References cited:
- WO-A2-2009/026604
- DE-A1- 4 227 586
- US-A- 5 027 303
- US-A1- 2007 027 631
- US-A1- 2007 129 769

## Description

### Subject of Invention

The subject of the invention, which is defined in independent claim 1 and the dependent claims, is a device for assessing and display of a difference in vectors of forces exercised by a pair of arms or legs. The device is preferably intended for exercising arms or legs of patients *suffering from a damage of the musculoskeletal or nervous system (e. g. hemiparesis).* The same device can be used by amateur *or* professional sportsmen to exercise a pair of arms *or* legs in order to achieve anticipated potential from the point of view of strength, speed and coordinated functioning of both limbs of the same pair. The device of the invention can further be used for playing computer games, in which a new element is added: a force vector actually produced by the player and not just simulated.

### Technical Problem

The technical problem solved by the present invention is a design of such device for exercising a *skeleton,* muscles and indirectly the nervous system that will enable a person, for instance a patient or sportsman to exercise without the presence of a trainer or physiotherapist and outside professional institutions. The device should provide for such exercises, in which a principle of therapy or sports should be taken into account. A success criterion of an individual exercise step, exercise or the entire exercising period should be constant and regular comparison of capability of one arm or leg with the same capability of another limb, *namely another limb of the exercising person or another person, e. g. therapist or trainer, who is supposed to perform an exercise technically properly and with adequate strength.* The device should regularly follow the mentioned principle and notify the exercising person about the success and provide advice how to proceed. In case of a therapy of a patient, the device should provide an ongoing assessment of the status of the healthy and affected limb, make a comparison and give the exercising person advice about the next exercising steps. The device should further forward these data to an expert for professional assessment and the expert should then provide the exercising person with detail instructions and can also use these data for professional research based on such information received from a multitude of exercising persons.

### Prior Art

Devices of this type are known from the field of rehabilitation and are described in US 5,027,303 (Witte) and US 2007/0027631 A1 (Normann). Such devices offer a patient a possibility of a variety of exercises for affected limbs, yet the ability of a healthy limb is never compared with the ability of a affected limb in any exercise and no instructions for further exercise are provided to a patient. The therapy for such purposes is carried out under a supervision of a therapist, who suggests changed exercises based on his professional assessment of treatment progress.

There is a need for a different device, the operation of which is based on the awareness of a physiological property that exercise or therapy of a affected limb is more efficient if the healthy limb is exercised simultaneously. A simultaneous exercise of the healthy and the affected limb of the same pair leads to a harmonious development of muscles and nervous system of the affected limb in comparison with the muscular and nervous situation of the healthy limb, the latter being an ongoing criterion for the strengthening of the affected limb. During exercise, the healthy limb can be a manipulator for a support of movement of the affected limb. The final result is a mutually balanced status of the muscular and nervous tissue and consequently of the entire healthy and healed limb of the same pair. In said exercise of the healthy and affected limb there is often a need for a therapist or trainer to participate with his healthy limb *and to direct the exercising of a patient's affected limb in a way that the exercising person feels the action of the therapist's or trainer's limb and tries to repeat it as properly and strongly as possible.*

### Solution to the Technical Problem

The described technical problem is solved by the device of the invention, the constructional characteristic and novelty of which is a personification of the described exercising principle in a way that the construction offers the exercising person such exercises and the exercising person does not need to be acquainted with these principles at all. The device of the invention offers and ensures proper exercising by allowing a simultaneous exercising of both limbs of the same pair, e. g. a healthy and a affected limb, and constantly compares the strength and direction of forces exercised by both limbs on the device, evaluates these data and provides the exercising person with a further instruction for exercising in the next step. *In analogy as described above, an adequate therapist's or trainer's limb is used. Further, theoretical knowledge and experience of a therapist are used, whose exercise provides the exercising person with a demonstration of proper performance and adequate strength of an exercise. The device of the invention constantly inputs various data about the exercise, so that subsequent* automatic operation of the device allows the exercising person *under the consideration of said input data* to exercise without a supervision of a professional and outside professional institutions and without a danger to exercise in a wrong or even harmful way. During an exercise, the device provides the exercising person with instructions in such audiovisual form and such content that the patient can include the instructions in his exercise without any previous knowledge.

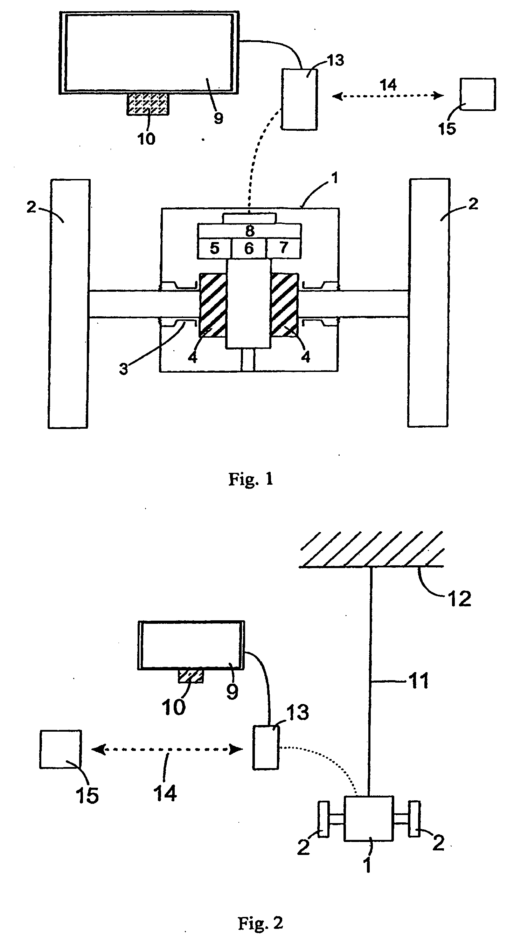

The essence of the invention will be explained by way of an embodiment and the enclosed drawing, representing in
Fig. 1 cross-section of the device of the invention according to the first embodiment,
Fig. 2 schematic view of the device of the invention according to the second embodiment, and

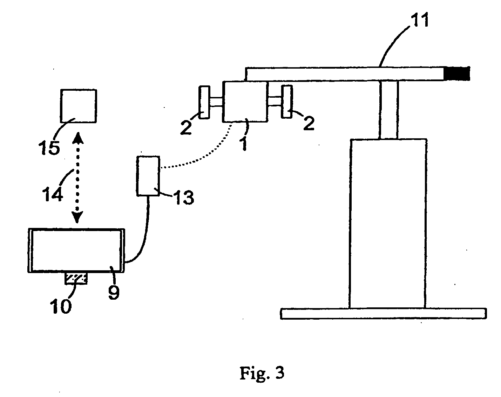

Fig. 3 schematic view of the device of the invention according to the third embodiment.

The device for assessing and display of a difference in vectors of forces exercised by a pair of arms or legs of exercising persons, e. g. patients suffering from hemiparesis, i. e. muscular disease of one body half, further of persons with damaged musculoskeletal system or sportsmen comprises a housing **1** with two means **2** for a participation with a pair of limbs - arms or legs that are not shown in figures. The patients are supposed to have only one limb affected with hemiparesis. The means **2** are designed in various ways with the purpose to allow proper participation with the treated pair of limbs in an individual exercise. It is therefore foreseen that the means **2** are interchangeable. The most common design of the means **2** is a handle hold by the exercising person with one hand. If affected limbs that are not capable of holding the means and exercising are in question, means **2** are foreseen that are mountable with known elements to physiotherapeutically adequate spot on limbs. The means **2** for legs are of a different design. A detail design of the means **2** is not the subject of the present invention.

The means **2** are arranged on bearings **3** within the housing **1**. Each means **2** is connected with a means **4** for the measurement of forces. The means **4** measures the force **F** that appears as a result of various activity of a limb exercised on the means **2** observable as bending. If both arms or legs are equally able, they can reach the same result in an exercise and the difference of forces amounts to 0, which *can be* the goal of such therapy or exercise. As the abilities of different exercising persons when healthy are different, the only proper way of assessing an exercise is a constant comparison of the ability of a healthy limb with that of the treated limb in the same exercise. *The healthy limb is thus a manipulator and the co-operation between both limbs is measured.*

Apart from those moves of individual limbs, the effect of which is measured by comparing the effect of one limb with respect to the other of the same pair, the exercise also includes those moves, in which a coordinated action of both limbs is required. It is anticipated that the healthy limb assists and guides the affected limb at the beginning of the therapy, until the affected limb gets strong enough, ideally equal to the healthy limb when the therapy is completed. Such exercise requires that the entire device of the invention should be moved with both limbs to prescribed directions.

The device of the invention is provided with additional sensors of position and orientation to monitor and assess these exercises, when the exercising person moves the device in space and simultaneously changes the speed of moves.

To determine direction of the force **F** an accelerometer **5**, a gyroscope **6** and a magnetometer **7** are used. The accelerometer **5**, the gyroscope **6** and the magnetometer **7** together assess the current orientation of the device in space with respect to the selected coordinate system. Details of this assembly are not the subject of the present invention.

Apart from measuring the orientation of the device its position needs to be measured in order to estimate the position of the device in space. A rough estimate can be obtained from the measurements by means of the accelerometer **5.** To obtain more precise measurements of the position of the device, a use of position sensors is foreseen. These sensors are based on mechanical principle, e. g. fastening of the device to a mechanism by means of joints and joint position sensors, optical principle, e. g. camera and markers or a video camera, ultrasound principle, e. g. use of ultrasound transmitters and receivers for the assessment of position, and electromagnetic principle, e. g. upgrade of the device with coils and arrangement of the device into a controlled electromagnetic field. It is possible to precisely determine the position of the device in space with respect to the selected coordinate system by means of orientation and position sensors.

All data from means **4**, accelerometer **5**, gyroscope **6** and magnetometer **7** are gathered by a microcontroller **8** that evaluates them with models and algorithms, which are not the subject of the present invention. The data are continuously displayed on a display **9**, a monitor or a TV screen, notifying the exercising person on the exercising status and suggesting next steps. The information can also be given in an audio way via speaker **10**, in addition to the visual information provided on the display **9**.

In the second embodiment an element **11** intended to connect the device of the invention to a selected fixed attachment **12** is added to said first embodiment. The connection of the device of the invention via element **11** to the attachment **12** is elastic with possibly also adjustable stiffness that offers the exercising person assistance, e.g. it for instance compensates for the weight of arms, or provides counter-resistance, e. g. simulates the force of gravity etc. as required by an individual exercise. The fixed attachment can be provided with a position sensor, which allows measuring the position of the device in space and partly substitutes the measurements performed with the devices described in the first two embodiments.

The element **11** in the third embodiment is upgraded within the meaning of a robot operated by a computer program and dictating and monitoring the controlled complex moves of the exercising person. The robot adds changing counteracting forces as dictated by each exercise to the exercising person. A robot of this type assists in a proper execution of exercises by guiding the exercising person to develop adequate force and its direction with the affected limb. The robot here moves the device of the invention as required by the exercise, whereas the exercising person follows or responds to the program by adequate movement of his exercising limbs with the anticipated force in the anticipated direction as required by the exercise. The robot measures the position in its joints and thus measures the position of the device in space.

The fourth embodiment is designed as an adaptation of the first embodiment. It is constructionally or rather dimensionally adapted to exercising one limb, especially a hand, wherein one limb, e. g. a hand, acts with one of its part, e.g. fingers on one means **2** and with the other part, e. g. palm to the other means **2**. One means **2** can also be mechanically grounded, wherein the device allows isometric exercising of the affected limb.

In all said embodiments a computer assembly is intended for operating the device of the invention. Said computer assembly receives data from said sensors of forces and accelerations and means for assessing spatial position of the device and its movement direction. These data are processed and stored by a microcontroller that constantly compares them with the preset values selected by the expert dictating the exercises or therapy. These values are target values of each individual exercise or the entire set of exercises. Of course, the computer assembly includes the foreseen models of each exercise that make up the basis for exercising carried out by this assembly. The data gathered from each exercise of an exercising person can be simultaneously or subsequently studied by experts. Statistical analysis of these data can serve as a proof of success of individual exercises. The device therefore comprises means for continuous forwarding of data to experts, e. g. network connection or for subsequent data transmission via optional known data carriers. The data are returned to the device by the same way - continuously or subsequently - in order to provide instructions for next exercises. The device of the invention thus enables an ongoing selection of exercises based on the speed and quality of rehabilitation of an individual exercising person.

Forwarding of instructions before and during an exercise and transmission of results after each exercise performed is carried out by means of a personal computer **13** (or adequate TV receiver) and the network connection **14**, via which the microcontroller **8** in the device of the invention and/or the physiotherapist receive data within the meaning of remote access to an expert centre (telerehabilitation) **15**, from where the exercising person receives adequate messages. Moreover, this centre **15** simultaneously gathers the data from various exercising persons and performs a professional processing.

As already mentioned earlier, the device of the invention is intended both for the therapy of affected persons and for the intended exercising of persons who want to harmonize the efficiency of a pair of limbs with respect to the current situation and needs. This is especially meant for sportsmen, especially top athletes, who may experience undesired mistakes in the development of the musculo-nervous tissues during exercise. It is herewith possible to harmonize the strength and reaction of a pair of limbs to act in harmony and with the same strength. It may even be that a sports branch requires correction of ability of an individual limb. This is all rendered possible by the device of the invention.

The device of the invention can further be used in computer games as a game interface. Currently known interfaces of this type (e. g. game consoles or joysticks) offer a player a possibility to submit various commands in analog and/or digital form and two- or three-directional control of one or several objects in a virtual space formed by the computer and displayed on a display. The device of the invention adds yet another playing dimension to the player: directed real force that the player creates on the device of the invention. The player uses this force to have influence on the course of the game as foreseen by the computer program of the game.

It is moreover self-evident to a man skilled in the art that the described device of the invention can be amended in a way to comprise more than just a pair of means for co-operation with a pair of limbs, e. g. for a game with more than one player or for more complex therapies that for instance not only act upon the musculo-nervous system of the limbs but also on other parts of the body.

The device of the invention further allows medical personnel to make an objective assessment of abilities of an exercising person in terms of coordination and range of motion, size and direction of deliberate forces and assessment of pathological indicators.

It is understandable that by being acquainted with the above description a man skilled in the art can design further embodiments without circumventing the characteristics of the invention defined in the appended claims.

## Claims

1. A device configured to assess and display a difference in vectors of forces exercised by a pair of arms or legs of exercising persons, like patients suffering from hemiparesis or sportsmen, comprising a housing **(1)** with two means **(2)** for a participation with a pair of limbs - arms or legs in an individual exercise, wherein said means **(2)** are arranged on bearings **(3)** within the housing **(1)**, further comprising a microcontroller **(8)**, and optionally a computer **(13)** with the network connection **(14)** for connection to an expert rehabilitation center **(15)**, **characterized in that** each means **(2)** is connected with a means **(4)** for the measurement of forces **(F)** that appear as a result of various activity of a limb exercised on the means, an accelerometer **(5)**, a gyroscope **(6)** and a magnetometer **(7)** for the determination of a position and an orientation of the device in space wherein all data from said means **(4)**, said accelerometer **(5)**, said gyroscope **(6)** and said magnetometer **(7)** are gathered by said microcontroller **(8)** and evaluated with models and algorithms, and the data are continuously displayed on a display **(9)**, a monitor or a TV screen, notifying the exercising person on exercising status and suggesting next steps.

2. Device according to Claim 1, **characterized in that** an element **(11)** intended to connect the device to a fixed attachment **(12)** is added, wherein said connection of the device via element **(11)** to the attachment **(12)** is elastic with possibly also adjustable stiffness.

3. Device according to Claim 1, **characterized in that** said element **(11)** is type of a robot operated by a computer program and dictates and monitors the controlled complex moves, adds changing counteracting forces as dictated by each exercise to the exercising person.

4. Device according to Claims 1 to 3, **characterized in that** a computer microcontroller **(8)** coordinates operation of the device, said microcontroller **(8)** receiving values transmitted by said sensors of forces and accelerations and means for assessing spatial position of the device and its movement direction, evaluating and storing them and constantly comparing them with the preset values selected by the expert dictating the exercises or therapy.

5. Device according to Claims **1** to **4**, **characterized in that** the means **(2)** are interchangeable in order to adapt to the treated limbs of an exercising person.

6. Device according to Claim 1, **characterized in that** it is adapted to exercising one limb, especially a hand, wherein one limb acts with one of its part on one means **(2)** and with the other part to the other means **(2).**

## Patentansprüche

1. Vorrichtung eingerichtet zum Beurteilen und Anzeigen einer Differenz in Vektoren von durch ein Paar Arme oder Beine von übenden Personen wie an Hemiparese leidenden Patienten oder Sportlern ausgeübten Kräften, umfassend ein Gehäuse (**1**) mit zwei Mitteln (**2**) für eine Teilnahme mit einem Paar Gliedern - Armen oder Beinen an einer einzelnen Übung, wobei diese Mittel (**2**) auf Lagern (**3**) innerhalb des Gehäuses (**1**) angeordnet sind, weiterhin umfassend eine Mikrosteuerung (**8**) und wahlweise einen Computer (**13**) mit der Netzverbindung (**14**) zur Verbindung mit einem Experten-Rehabilitationszentrum (**15**), **dadurch gekennzeichnet, dass** jedes Mittel (**2**) mit einem Mittel (**4**) zur Messung von Kräften (**F**), die als ein Ergebnis verschiedener der auf den Mitteln ausgeübter Tätigkeit eines Gliedes erscheinen, einem Beschleunigungsmesser (**5**), einem Kreisel (**6**) und einem Magnetometer (**7**) zur Bestimmung einer Position und einer Orientierung der Vorrichtung im Raum, wobei alle Daten von diesen Mitteln (**4**), dem Beschleunigungsmesser (**5**), dem Kreisel (**6**) und dem Magnetometer (**7**) durch die Mikrosteuerung (**8**) eingesammelt und mit Modellen und Algorithmen ausgewertet werden, und die Daten fortlaufend auf einer Anzeige (**9**), einem Monitor oder einem Fernsehbildschirm angezeigt werden und die übende Person über den Übungszustand benachrichtigen und nächste Schritte vorschlagen.

2. Vorrichtung nach Anspruch 1, **dadurch gekennzeichnet, dass** ein Element (**11**)**,** das die Vorrichtung mit einer festen Halterung (**12**) verbinden soll, hinzugefügt wird, wobei diese Verbindung der Vorrichtung über Element (**11**) mit der Halterung (**12**) mit möglicherweise auch einstellbarer Steifheit elastisch ist.

3. Vorrichtung nach Anspruch 1, **dadurch gekennzeichnet, dass** das Element (**11**) eine Art Roboter betrieben durch ein Computerprogramm ist und die gesteuerten komplexen Bewegungen diktiert und überwacht, veränderliche gegenwirkende Kräfte wie durch jede Übung diktiert der übenden Person zufügt.

4. Vorrichtung nach Ansprüchen 1 bis 3, **dadurch gekennzeichnet, dass** eine Computermikrosteuerung (**8**) den Betrieb der Vorrichtung koordiniert, wobei die Mikrosteuerung (**8**) durch die Sensoren übertragene Werte von Kräften und Beschleunigungen und von Mitteln zum Beurteilen der räumlichen Lage der Vorrichtung und ihrer Bewegungsrichtung empfängt, sowie Auswerten und Speichern derselben und fortlaufendes Vergleichen derselben mit dem durch den die Übungen oder die Therapie diktierenden Experten ausgewählten voreingestellten Werten.

5. Vorrichtung nach Ansprüchen 1 bis 4, **dadurch gekennzeichnet, dass** die Mittel (**2**) austauschbar sind, um sich an die behandelten Glieder einer übenden Person anzupassen.

6. Vorrichtung nach Anspruch 1, **dadurch gekennzeichnet, dass** sie angepasst ist zum Üben eines Gliedes, besonders einer Hand, wobei ein Glied mit einem Teil auf einem Mittel (**2**) wirkt und mit dem anderen Teil am anderen Mittel (**2**).

## Revendications

1. Dispositif configuré pour évaluer et afficher une différence de vecteurs de forces exercées par une paire de bras ou de jambes de personnes s'exerçant, comme des patients souffrant d'hémiparésie ou des sportifs, comportant un boîtier (1) doté de deux moyens (2) servant à une participation avec une paire de membres - bras ou jambe- à un exercice individuel, lesdits moyens (2) étant disposés sur des paliers (3) à l'intérieur du boîtier (1), comportant en outre un microcontrôleur (8), et éventuellement un ordinateur (13) doté d'une connexion (14) à un réseau pour se connecter à un centre expert (15) de rééducation, **caractérisé en ce que** chaque moyen (2) est relié à un moyen (4) servant à la mesure de forces (F) qui apparaissent en raison de diverses activités d'un membre exercé sur les moyens, un accéléromètre (5), un gyroscope (6) et un magnétomètre (7) servant à la détermination d'une position et d'une orientation du dispositif dans l'espace, toutes les données provenant dudit moyen (4), dudit accéléromètre (5), dudit gyroscope (6) et dudit magnétomètre (7) étant collectées par ledit microcontrôleur (8) et évaluées à l'aide de modèles et d'algorithmes, et les données étant affichées en continu sur un affichage (9), un moniteur ou un écran de TV, informant la personne s'exerçant de l'état d'exercice et suggérant des étapes suivantes.

2. Dispositif selon la revendication 1, **caractérisé en ce qu'**un élément (11) destiné à relier le dispositif à une implantation fixe (12) est ajouté, ladite liaison du dispositif via un élément (11) à l'implantation (12) étant élastique et présentant également éventuellement une rigidité réglable.

3. Dispositif selon la revendication 1, **caractérisé en ce que** ledit élément (11) est du type d'un robot manoeuvré par un programme d'ordinateur et **en ce qu'**il impose et surveille les mouvements complexes commandés, ajoute des forces antagonistes évolutives telles qu'imposées par chaque exercice à la personne s'exerçant.

4. Dispositif selon les revendications 1 à 3. **caractérisé en ce qu'**un microcontrôleur (8) d'ordinateur coordonne le fonctionnement du dispositif, ledit microcontrôleur (8) recevant des valeurs émises par lesdits capteurs de forces et d'accélérations et les moyens d'évaluation de la position spatiale du dispositif et de son sens de déplacement, les évaluant et les stockant et les comparant constamment aux valeurs prédéfinies sélectionnées par l'expert implosant les exercices ou la thérapie.

5. Dispositif selon les revendications 1 à 4, **caractérisé en ce que** les moyens (2) sont interchangeables afin de s'adapter aux membres traités d'une personne s'exerçant.

6. Dispositif selon la revendication 1, **caractérisé en ce qu'**il est prévu pour exercer un membre, en particulier une main, un membre agissant avec une de ses parties sur un moyen (2) et avec l'autre partie sur l'autre moyen (2).
